# EUROPEAN PATENT SPECIFICATION

(11) **EP 1 664 263 B1**
(45) Date of publication and mention of the grant of the patent: **09.04.2014**
(21) Application number: 04768536.7
(22) Date of filing: 20.09.2004
(51) Int. Cl.: B01L 3/02, C12M 1/32, G01N 35/10

(54) **APPARATUS FOR PROCESSING BIOLOGICAL MATERIALS**
VORRICHTUNG ZUR BEARBEITUNG BIOLOGISCHER MATERIALIEN
APPAREIL POUR TRAITER DES MATIERS BIOLOGIQUES

(30) Priority: 20.09.2003 GB 0322089
(43) Date of publication of application: 07.06.2006
(73) Proprietor: Mast Group Limited, Bootle, Merseyside L20 1EA (GB)
(72) Inventor: WILLIAMS ,David, Hugh, Liverpool, Merseyside L21 0JH (GB)
(74) Representative: Read, David Graham
(86) International application number: PCT/GB2004/003990
(87) International publication number: WO 2005/028609

(56) References cited:
- GB-A- 2 247 076
- US-A- 4 235 971
- US-A- 5 314 825

## Description

The present invention relates to an apparatus for processing biological materials.

The method of performing a standardized experiment on a number of biological samples is not only time consuming, but also requires a great deal of accuracy in order that the correct results are obtained. An example of an experimental protocol which is time consuming is the testing of biological samples for the presence of a microbial agent such as a strain of bacteria. Usually, the laboratory worker would usually perform a number of experiments from one biological sample which requires inoculating fresh reagents with the biological sample or alternatively, testing a number of samples with the same reagent contained in different vessels. If the experiment is to ascertain whether an individual is infected with a micro-organism, false positives or indeed false negatives could potentially be fatal. Therefore, most experiments are conducted in duplicate or triplicate in order to eliminate any human error, thus increasing the time required to perform such experiments. Furthermore, using reagents which have been made up in a slightly different manner may also produce spurious results.

In order to address the problems associated with human error and the amount of time taken to perform experiments on multiple samples, a number of devices have been developed. Most devices rely upon independent movements derived from a mechanical source in order to transfer the inoculum to a test surface such as a plate or a petri dish. Such devices rely upon a vertical motion for the inoculating head and a sliding motion for the plates or alternatively a vertical motion for the head and a rotary motion for the plates. Such reliance upon multiple movements of the head and the plates require a device employing complicated mechanical parts in order to actuate the movement which are complex and expensive to service and often require calibration prior to use.

GB 2247076 discloses a multi-inoculation apparatus for the semi-automated inoculation of a series of petri dishes or microtitre trays which has an inoculator head that is carried on a piston so that it can be moved up and down whilst the petri dishes may be moved into contact with the head and may additionally move in a rotational manner. Whilst the apparatus greatly reduces the time required to perform experiments on multiple biological samples, it relies upon the accuracy of the laboratory user in order maneuver the plates into a suitable position prior to inoculation. Furthermore, this apparatus also requires a number of movements in different orientations in order to achieve the desired results.

US 4, 235, 971 discloses an inoculator wherein a multiplicity of small volumes of an inoculant are picked up on pins depending from an inoculator head and deposited in separate areas according to a pattern, such as in the walls of a microtitration tray. This device requires the movement of the inoculating pins in a vertical and also horizontal manner in order to inoculate reagents disposed on a microtitration tray. Therefore, whilst this device reduces the amount of time required to perform multiple experiments on a number of biological samples, the requirement for the inoculating head to move vertically and horizontally requires a complex mechanism in order to arcuate its movement.

It is an object of the present invention to alleviate more and more of the aforementioned problems associated with the prior art. It is also an object of the present invention to provide an apparatus which can be used to process biological materials efficiently and accurately. Furthermore, it is also an object of the present invention to provide an apparatus which has a single movement in order to transfer biological samples to a testing area so as to reduce the cost of producing the apparatus and to assist the maintenance of the apparatus.

In accordance with the present invention, there is provided an apparatus for processing biological materials, the apparatus comprising a head with a transfer means disposed thereon and further comprising a first discrete area for holding a plate with one or more biological samples and a second discrete area holding a plate with one or more constituents used to process the biological samples, the head being operatively coupled to a shaft which moves in an arcuate manner so as to facilitate the transfer of material between plates. Therefore, the apparatus allows for the material to be transferred between plates (or indeed petri dishes or microtitre trays) with a single movement which reduces the need for complex mechanical parts and also permits accurate transfer of material between plates.

The head may comprise a plurality of transfer means. Preferably, the transfer means comprises a pin, a capillary tube or a wire loop. It will be evident, that a cleaning or sterilization means to clean or sterilize the transfer means may be necessary in order to prevent cross-contamination between different experiments. Such a cleaning or sterilization means may comprise a heating element to heat the pin or wire loop or alternatively a sterilizing solution in order to neutralize any contaminants. Preferably, the head is removable in order to facilitate cleaning and servicing and may further permit the interchanging of different sized or configured heads (for example the number of transfer means. Furthermore, it would be most desirable if the head were capable of being auto claved. The transfer means may be able to hold part of the sample by means of surface tension or capillary action in the case that the sample is in a liquid form or alternatively it would rely upon picking up an amount of solid material by means of friction. The pin may be of a standard size in order to comply with NCCLS (National Committee for Clinical Laboratory Standards) procedures. Preferably the pin will have a 0.3µl or 1 µl drop pin size.

Preferably, the head is maintained substantially horizontal during its movement. Furthermore, the head may be operatively coupled to a horizontal shaft.

The plates may comprise a plurality of wells disposed thereon, each well being separate from one another. Therefore, the head can interface with the sample and the constituents to a location which corresponds to one another. Therefore, a sample from well 9 can be used to inoculate well 9 of the plate holding the constituents for processing biological material. The number of wells disposed on a given plate may be in the range of 2 to 200. Preferably, the plate is a standardized plate with 19, 36, 96 or 100 wells disposed thereon. It will be evident to one skilled in the art, that the term "plates" also refer to petri-dishes, or microtitre trays or similar apparatus for performing multiple experiments or biological experiments. The plates may remain substantially static during the operation of the apparatus, although they may be replaced after operation.

The biological samples may comprise a number of different materials or sample types, including solid and liquid suspensions. Preferably, the biological sample is a preparation of one or more micro-organisms and such a preparation may originate from an individual. Therefore, the individual may be tested by a number of constituents or reagents in order to identify which micro-organisms are present within the sample. Preferably, the apparatus is used to inoculate a plate held in the second area with biological material from a plate held in the first area. The apparatus may perform its function by means of a cycle which is defined by the movement of the head from the first area to the second area and back again. Preferably, the apparatus further comprises a counter in order to ascertain the number of cycles performed.

The movement of the head may be controlled by means of a central processing unit (a CPU). Furthermore, the movement of the head may be subject to an adjustable time delay which may or may not be controlled by a CPU. The movement of the head may be controlled by a switch on the apparatus which may additionally define the time delay required. Alternatively, the head may be controlled by a foot pedal. The position of the head may be sensed by at least one sensor and the information from the sensor may be relayed to the CPU in order to allow the head to be positioned relative to the plates. The position of the head relative to the plates may also be adjustable in order to account for variations on the plate. It will be apparent to one skilled in the art, that such variations may be the total depth of growth media used on a plate or the quantity of the constituent used for the reaction. The position of the head relative to the plate may be automatically adjustable and this may be by means of the information relayed from the sensor to the CPU. Preferably, during the movement of the head, the acceleration and deceleration of the head is controlled in order to prevent the biological sample from falling from the transfer means.

The apparatus may be powered by a 12 volts direct current external power supply and may be capable of operating within the voltages of 100- 240 alternating current and between the frequencies of approximately 47-63Hz. It will be apparent, that this will allow the apparatus to be used in a number of countries without modification to the power source.

The discrete area for holding a plate may be a platform which substantially conforms to the shape of a plate, petri-dish or microtitre tray. Furthermore, the platform may be removable and/or reversible so as to accommodate a number of different shaped plates.

The apparatus therefore provides a means of processing biological materials which does not require any calibration as the heads relative position to the plates will always remain the same and it will therefore only require adjustment of the height of the head away from the plates (which will accommodate for variations in plate type and the material prepared thereon).

The present invention will now be described, by way of example only, with reference to the accompanying Figure:-
Fig. 1 is a perspective view of an apparatus in accordance with the present invention; and
Figs. 2A-C are diagrams of the mechanism by which the head moves in accordance with the present invention.

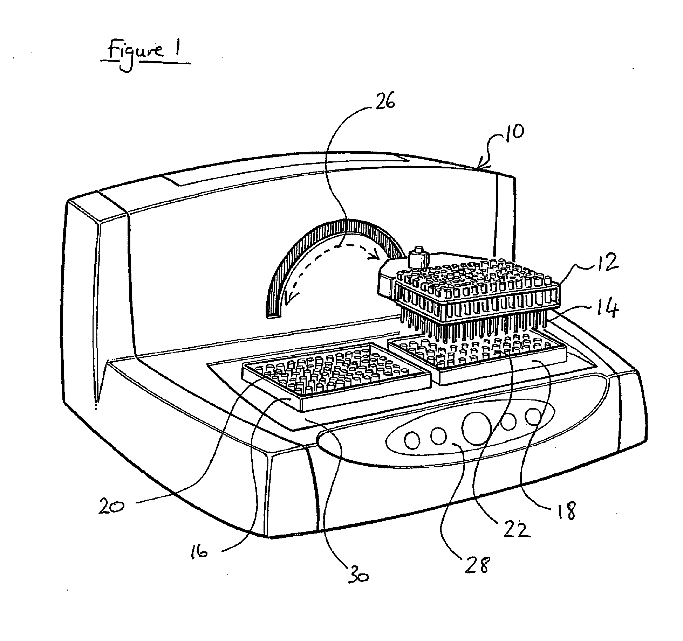

Figure 1 provides an apparatus 10 for processing biological materials comprising a head 12 which can move in an arcuate manner in directions 26. The head 12 has a plurality of pins 14 disposed thereon in order to transfer biological material from a first petri dish 16 to a second petri dish 18 (please note that references to "petri dishes" also refers to plate and microtitre trays). Each petri dish has a number of wells 20 and 22 which can hold biological samples and house biological reactions respectively. The head 12 can allow material to be transferred between both plates by moving in an arcuate manner along a slot 24 which is cut in the main body of the apparatus 10. The slot 24 is small in order to prevent contaminants from entering the mechanism of the apparatus 10 and also to minimise harm to a laboratory user. The slot 24 can additionally have a cover in place in order to prevent material from entering the mechanism. The apparatus also has a control panel 28 to operate the movement of the head 12 and a platform 30 to hold the petri-dishes.

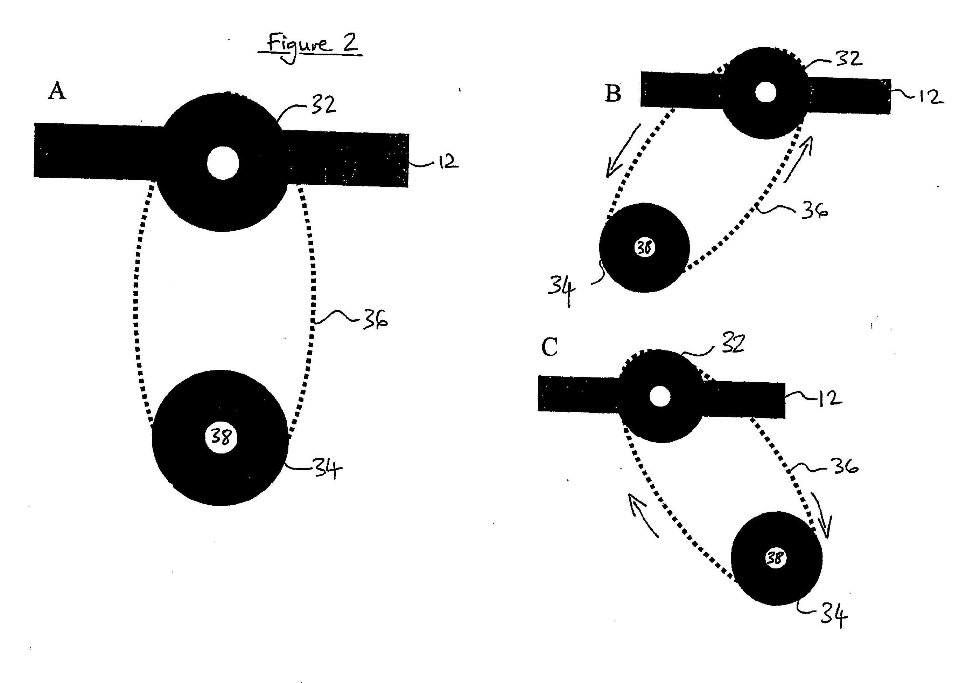

Figs. 2A-C provide a mechanism which can be employed in order to ensure that the head 12 maintains in a substantially horizontal position throughout its arcuate movement. The head 12 is operatively coupled to an upper pulley 32 which can receive a toothed belt 36 which in turn is connected to a pulley on the central drive shaft 38. The head 12 is kept substantially horizontal by the rotation of the drive shaft 38 which can rotate in either direction. The arrows in Fig. 2B and Fig. 2C show that the rotation of the shaft 38 in either an anti-clockwise or clockwise direction respectively will result in the head 12 being kept substantially horizontal despite the position of the head 12 throughout its arcuate path through the slot 24.

In use, inoculum in the form of either a sample suspension or solid matter is disposed in the wells 20 on the first petri dish 16 and the head 12 moves to a position over the petri dish 16 and allows the pins 14 to collect a quantity of inoculant. The head 12 then moves in an arcuate fashion along the slot 24 (whilst the head remains substantially horizontal) and then deposits the inoculum into a corresponding well 22 on a petri dish 18 which contains reagents or further processing constituents. The head 12 then reverts back to the first petri dish 16 along the arcuate slot 24 along the direction 26. A laboratory user can then remove the second petri dish 18 and replace it with a further petri dish should further inoculations be required.

The apparatus will operate using a single 12 volt direct current external power supply which will allow the apparatus to work on most main voltages between 100 and 240 volts (alternating current) and frequencies between 47 and 63 Hz.. Therefore, the apparatus can be used in most locations without any modifications.

The apparatus can also employ software to control the acceleration and deceleration of the drive motor which powers the head 12 in order to obtain smooth starting and stopping and minimizing inoculum "drop-off" which is a commonly reported problem with prior art devices. The apparatus can have a number of operational modes but will commonly have two: a single cycle with one operational mode which can be initiated by a start button on the front of the panel 28 or by a foot switch (not shown): or a continuous cycle can also be employed which once started, the operation is repeated until the stop button is pressed at which point the current cycle is completed before the head 12 is stopped. In a continuous cycle mode, a delay can also be introduced between cycles in order to allow extra time for the operator to change the second petri dish 18 and the delay can be set from anything between 0 and 10 seconds (having 0.5 second incrementation).

The head 12 is connected to a central shaft via an arm (not shown in the Figure) which can ensure an accurate and repeatable inoculum placement from the first petri dish 16 to the second petri dish 18 and the lateral placement of the inoculum is dependent only upon the distance between the arm and head 12 which contains the pins 14.

The head 12 is counter balanced in order to minimise the difference in speed between the head-raising and head-lowering parts of the cycle. The head 12 can be fitted either way up which allows the pins 14 to be inserted in either orientation and the head will usually have the same shape and size and configuration to allows but can be configured to accommodate bespoke petri dishes. Furthermore, a head which conforms to standard sizes of petri dish and plate can be used in order to allow the petri dishes/plates to be used in other machines and/or protocols. Commonly, the heads will contain a 19,36,96 or 100 pins 14 which will conform to petri dishes 16 and 18 with the same number of wells 20 and 22. For the 19 and 36 pin head, a ballast weight is also included which minimizes the difference in weight from the 96 and 100 pin heads. This ballast can also contain a key way which prevents rotation of the knife-edge marker used on circular dishes should they be used.

The arm can rotate independently form the central shaft by means of a collar which is attached to the head. Therefore the rotation of the head can be adjusted relative to actual position of the shaft in the slot 24.

The head 12 is kept substantially horizontal throughout its arcuate path 26 using two toothed pulleys 32 and 34 and a toothed belt 36. A central pulley 32 may be mounted on a chassis in line with the central shaft 38 which passes through a bearing in the pulley which serves to center the pulley 32 about the central shaft. The pulleys orientation can be adjusted to set the head 12 to a horizontal position and is prevented from rotating by two mounting screws. The outer pulley on the head 12 arm is fixed to the shaft and is attached at the other end of the shaft so that the whole outer shaft assembly is free to rotate in the slot 24. The toothed belt 36 is installed between the two pulleys and this combination has a similar effect as a 4-bar parallelogram linkage mechanism.

The operation can be described as follows: - As the arm rotates clockwise, the belt winds onto the left of the central pulley and unwinds off the right side. This causes the belt to wind on the right hand side of the outer pulley and off the left side by the same amount thus maintaining the same angular orientation of the two pulleys and, therefore the horizontal orientation of the head at all points of the arc.

The position of the head 12 can be sensed by three optical switches (one at each end of travel and having adjustable heights to suit the levels of inoculum and recipient surface on both petri dishes 16 and 18) and one on the motor drive shaft 38 which provides eight pulses per revolution and is therefore used to determine the intermediate positions of the head 12. The software used by the apparatus 10 employs the pulse count to set the point at which the head 12 is stopped during the normal operation and also controls the load position and the speed transition points of the head 12 during its motion. The position count can be reset each time the arm reaches the inoculum pickup point in order to prevent cumulative error.

Commonly, the arm is driven by a worm and wheel arrangement (not shown) which provides a gearing reduction from the motor speed and also prevents unwanted movement of the arm through the application of external forces, although a number of different methods of actuating movement of the arm about the arc may be utilised in place of a worm and wheel arrangement. Furthermore, the worm and wheel arrangement may be coupled to the drive shaft 38 so that both the movement of the arm through its arcuate path and the action to maintain the horizontal position of the head 12 are linked.

The platform on which the petri dishes or plates are placed can be reversible in order to allow for different types of specimen plate/dish to be used and may be adjustable in order to allow for any different in height of the growth medium in these plates. The platform 30 is held within the apparatus 10 by means of mounting holes and locating pins for easy adjustment and/or reversal.

A cover in the form of a disc may be attached to the front of the central shaft just behind the slot 24 and the arm in order to minimize any ingress of moisture during cleaning and to prevent injury to users who may try to place their fingers in the slot. Furthermore, an electro mechanical seven-digit counter can be fitted to the rear panel of the apparatus 10 in order to indicate the number of inoculations cycles that the unit has performed. Therefore, servicing regimes can be adhered to or the use of the device monitored by companies etc.

## Claims

1. An apparatus for processing biological materials, the apparatus comprising a head with a transfer means disposed thereon and further comprising a first discrete area for holding a plate with one or more biological samples and a second discrete area holding a plate with one or more constituents used to process the biological samples, the head being operatively coupled to a shaft which moves in an arcuate manner so as to facilitate the transfer of material between plates.

2. An apparatus as claimed in claim 1, wherein the head comprises a plurality of transfer means.

3. An apparatus as claimed in either claim 1 or claim 2, wherein the transfer means comprises a pin, a capillary tube or a wire loop.

4. An apparatus as claimed in any preceding claim, wherein the transfer means further comprises a cleaning or sterilization means.

5. An apparatus as claimed in claim 4, wherein the cleaning or sterilization means comprises a heating element or a sterilizing solution.

6. An apparatus as claimed in any preceding claim, wherein the head is removable.

7. An apparatus as claimed in any of claims 1 to 5, wherein the head is interchangeable with differently sized or configured heads.

8. An apparatus as claimed in any preceding claim, wherein the head is capable of being auto claved.

9. An apparatus as claimed in any preceding claim, wherein the transfer means is capable of holding part of the sample by means of surface tension or capillary action.

10. An apparatus as claimed in any preceding claim, wherein the transfer means comprises a pin.

11. An apparatus as claimed in any preceding claim, wherein the head is maintained substantially horizontal during its movement

12. An apparatus as claimed in any preceding claim, wherein the head is operatively coupled to a horizontal shaft.

13. An apparatus as claimed in any preceding claim, wherein the plate has a plurality of wells disposed thereon.

14. An apparatus as claimed in claim 13, wherein the number of wells disposed on a given plate is in the range of 2 to 200.

15. An apparatus as claimed in any preceding claim, wherein the plates remain substantially static during the operation of the apparatus.

16. An apparatus as claimed in any preceding claim, wherein the apparatus is used to inoculate a plate held in the second area with biological material from a plate held in the first area.

17. An apparatus as claimed in any preceding claim, wherein apparatus performs its function by means of a cycle which is defined by the movement of the head from the first area to the second area and back again.

18. An apparatus as claimed in any preceding claim, wherein the movement of the head is controlled by means of a central processing unit.

19. An apparatus as claimed in any preceding claim, wherein the movement of the head is subject to an adjustable time delay.

20. An apparatus as claimed in any preceding claim, wherein the position of the head is sensed by at least one sensor.

21. An apparatus as claimed in any preceding claim, wherein the position of the head relative to the plate is adjustable.

22. An apparatus as claimed in any preceding claim, wherein the acceleration and deceleration of the head is controlled in order to prevent the biological sample from falling from the transfer means.

23. An apparatus as claimed in any preceding claim, wherein the discrete area for holding a plate substantially conforms to the shape of a plate, petri-dish or microtitre tray.

## Patentansprüche

1. Vorrichtung zum Verarbeiten von biologischen Materialien, wobei die Vorrichtung einen Kopf mit einem daran angeordneten Transfermittel umfasst und weiter einen ersten diskreten Bereich zum Halten einer Platte mit einer oder mehreren biologischen Proben und einen eine Platte mit einem oder mehreren zum Verarbeiten der biologischen Proben verwendeten Bestandteilen verwendeten haltenden zweiten diskreten Bereich umfasst, wobei der Kopf wirksam an einen Schaft gekoppelt ist, der sich auf bogenförmige Weise bewegt, um den Transfer von Material zwischen Platten zu ermöglichen.

2. Vorrichtung nach Anspruch 1, wobei der Kopf mehrere Transfermittel umfasst.

3. Vorrichtung nach Anspruch 1 oder Anspruch 2, wobei das Transfermittel einen Zapfen, eine Kapillarröhre oder eine Drahtschlaufe umfasst.

4. Vorrichtung nach einem der vorangehenden Ansprüche, wobei das Transfermittel weiter ein Reinigungs- oder Sterilisationsmittel umfasst.

5. Vorrichtung nach Anspruch 4, wobei das Reinigungs- oder Sterilisationsmittel ein Heizelement oder eine Sterilisationslösung umfasst.

6. Vorrichtung nach einem der vorangehenden Ansprüche, wobei der Kopfabnehmbar ist.

7. Vorrichtung nach einem der Ansprüche 1 bis 5, wobei der Kopf mit verschieden bemessenen oder ausgebildeten Köpfen austauschbar ist.

8. Vorrichtung nach einem der vorangehenden Ansprüche, wobei der Kopf autoklaviert werden kann.

9. Vorrichtung nach einem der vorangehenden Ansprüche, wobei das Transfermittel in der Lage ist, einen Teil der Probe mittels Oberflächenspannung oder Kapillarwirkung zu halten.

10. Vorrichtung nach einem der vorangehenden Ansprüche, wobei das Transfermittel einen Zapfen umfasst.

11. Vorrichtung nach einem der vorangehenden Ansprüche, wobei der Kopf während seiner Bewegung im Wesentlichen horizontal gehalten wird.

12. Vorrichtung nach einem der vorangehenden Ansprüche, wobei der Kopf wirksam an einen horizontalen Schaft gekoppelt ist.

13. Vorrichtung nach einem der vorangehenden Ansprüche, wobei die Platte mehrere darauf angeordnete Näpfe aufweist.

14. Vorrichtung nach Anspruch 13, wobei die Zahl der auf einer gegebenen Platte angeordneten Näpfe im Bereich von 2 bis 200 liegt.

15. Vorrichtung nach einem der vorangehenden Ansprüche, wobei die Platten während des Betriebs der Vorrichtung im Wesentlichen unbewegt bleiben.

16. Vorrichtung nach einem der vorangehenden Ansprüche, wobei die Vorrichtung verwendet wird, um eine in dem zweiten Bereich gehaltene Platte mit biologischem Material von einer in dem ersten Bereich gehaltenen Platte zu impfen.

17. Vorrichtung nach einem der vorangehenden Ansprüche, wobei die Vorrichtung ihre Funktion mittels eines Zyklus ausführt, der von der Bewegung des Kopfs von dem ersten Bereich zu dem zweiten Bereich und wieder zurück definiert wird.

18. Vorrichtung nach einem der vorangehenden Ansprüche, wobei die Bewegung des Kopfs mittels einer zentralen Verarbeitungseinheit gesteuert wird.

19. Vorrichtung nach einem der vorangehenden Ansprüche, wobei die Bewegung des Kopfs einer verstellbaren Zeitverzögerung unterliegt.

20. Vorrichtung nach einem der vorangehenden Ansprüche, wobei die Lage des Kopfs von mindestens einem Sensor abgefühlt wird.

21. Vorrichtung nach einem der vorangehenden Ansprüche, wobei die Lage des Kopfs relativ zu der Platte verstellbar ist.

22. Vorrichtung nach einem der vorangehenden Ansprüche, wobei die Beschleunigung und Verzögerung des Kopfs gesteuert werden, um zu verhindern, dass die biologische Probe von dem Transfermittel fällt.

23. Vorrichtung nach einem der vorangehenden Ansprüche, wobei die Form des diskreten Bereichs zum Halten einer Platte im Wesentlichen der From einer Platte, Petrischale oder Mikrotiterplatte entspricht.

## Revendications

1. Appareil permettant de traiter des matières biologiques, l'appareil comportant une tête ayant un moyen de transfert disposé sur celle-ci et comportant par ailleurs une première zone discrète destinée à retenir une plaque ayant un ou plusieurs échantillons biologiques et une seconde zone discrète retenant une plaque ayant une ou plusieurs parties constitutives servant à traiter les échantillons biologiques, la tête étant accouplée de manière fonctionnelle à une tige qui se déplace d'une manière arquée afin de faciliter le transfert de matière entre les plaques.

2. Appareil selon la revendication 1, dans lequel la tête comporte une pluralité de moyens de transfert.

3. Appareil selon la revendication 1 ou la revendication 2, dans lequel le moyen de transfert comporte une broche, un tube capillaire ou une boucle de fil métallique.

4. Appareil selon l'une quelconque des revendications précédentes, dans lequel le moyen de transfert comporte par ailleurs un moyen de nettoyage ou de stérilisation.

5. Appareil selon la revendication 4, dans lequel le moyen de nettoyage ou de stérilisation comporte un élément de chauffage ou une solution de stérilisation.

6. Appareil selon l'une quelconque des revendications précédentes, dans lequel la tête est amovible.

7. Appareil selon l'une quelconque des revendications 1 à 5, dans lequel la tête est interchangeable avec des têtes de différente taille ou configuration.

8. Appareil selon l'une quelconque des revendications précédentes, dans lequel la tête est en mesure d'être autoclavée.

9. Appareil selon l'une quelconque des revendications précédentes, dans lequel le moyen de transfert est en mesure de retenir une partie de l'échantillon par tension superficielle ou action capillaire.

10. Appareil selon l'une quelconque des revendications précédentes, dans lequel le moyen de transfert comporte une broche.

11. Appareil selon l'une quelconque des revendications précédentes, dans lequel la tête est maintenue de manière sensiblement horizontale lors de son mouvement.

12. Appareil selon l'une quelconque des revendications précédentes, dans lequel la tête est accouplée de manière fonctionnelle à une tige horizontale.

13. Appareil selon l'une quelconque des revendications précédentes, dans lequel la plaque a une pluralité de compartiments disposés sur celle-ci.

14. Appareil selon la revendication 13, dans lequel le nombre de compartiments disposés sur une plaque donnée est de l'ordre de 2 à 200.

15. Appareil selon l'une quelconque des revendications précédentes, dans lequel les plaques restent sensiblement statiques lors du fonctionnement de l'appareil.

16. Appareil selon l'une quelconque des revendications précédentes, dans lequel l'appareil sert à inoculer une plaque retenue dans la seconde zone avec de la matière biologique en provenance d'une plaque retenue dans la première zone.

17. Appareil selon l'une quelconque des revendications précédentes, dans lequel l'appareil réalise sa fonction selon un cycle qui est défini par le mouvement de la tête depuis la première zone jusqu'à la seconde zone et de retour.

18. Appareil selon l'une quelconque des revendications précédentes, dans lequel le mouvement de la tête est commandé par le biais d'une unité centrale de traitement.

19. Appareil selon l'une quelconque des revendications précédentes, dans lequel le mouvement de la tête est soumis à une temporisation réglable.

20. Appareil selon l'une quelconque des revendications précédentes, dans lequel la position de la tête est détectée par au moins un capteur.

21. Appareil selon l'une quelconque des revendications précédentes, dans lequel la position de la tête par rapport à la plaque est réglable.

22. Appareil selon l'une quelconque des revendications précédentes, dans lequel l'accélération et la décélération de la tête sont régulées afin d'empêcher l'échantillon biologique de tomber du moyen de transfert.

23. Appareil selon l'une quelconque des revendications précédentes, dans lequel la zone discrète servant à retenir une plaque correspond sensiblement à la forme d'une plaque, d'une boîte de Petri ou d'un plateau à micro-titration.
